(19) Europäisches Patentamt
European Patent Office
Office européen des brevets

(11) Publication number: 0 348 170 A2

(12) **EUROPEAN PATENT APPLICATION**

(21) Application number: 89306259.6

(22) Date of filing: 21.06.89

(51) Int. Cl.[4]: C 07 H 19/06
C 07 H 19/09, C 07 H 19/073,
A 61 K 31/70, C 07 D 405/04,
A 61 K 31/505, C 07 F 7/18

(30) Priority: 21.06.88 US 209570

(43) Date of publication of application:
27.12.89 Bulletin 89/52

(84) Designated Contracting States:
AT BE CH DE ES FR GB GR IT LI LU NL SE

(71) Applicant: Lee, Moses Nam Fong
3464 Hillview Crescent
Edmonton Alberta T6L 1P5 (CA)

(72) Inventor: Lee, Moses Nam Fong
3464 Hillview Crescent
Edmonton Alberta T6L 1P5 (CA)

(74) Representative: Pidgeon, Robert John et al
Appleyard Lees & Co. 15 Clare Road
Halifax West Yorkshire HX1 2HY (GB)

(54) **Antiviral pyrimidine derivatives.**

(57) A 1-(3′-substituted-2′,3′-didehydro-2′,3′-dideoxyribofuranosyl)pyrimidine derivative of the general formula I:

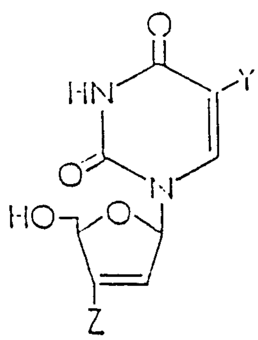

.....I

is provided wherein:
Y is H, or a $C_1$-$C_6$ alkyl group; and
Z is an azido group or a 1-(1,2,3-triazolo) group, or a cyano group, or an optionally substituted alkynyl group, or an optionally substituted alkenyl group, or an optionally substituted sulfide group, or an optionally substituted sulfone group.

EP 0 348 170 A2

**Description**

## ANTIVIRAL PYRIMIDINE DERIVATIVES

### TECHNICAL FIELD OF THE INVENTION

The invention relates to novel 1-(3′-substituted -2′,3′-didehydro-2′,3′-dideoxyribofuranosyl)pyrimidine derivatives and to their use primarily as antiviral agents, specifically anti-AIDS agents.

### BACKGROUND OF THE INVENTION

Nucleoside analogues are currently being widely investigated as potential antiviral agents against the human immunodeficiency virus (HIV), the etiologic agent of AIDS. [J. Med. Chem. 30, 1270-1278 (1987); ibid 31, 336-340 (1988)].

Examples of highly potent anti-HIV pyrimidine nucleosides comprise 3′-azido-3′-deoxythymidine (AZT); 3′-azido-2′,3′-dideoxy-5-bromouridine (DBU); 3′-azido-2,3′-dideoxy-5′-iodouridine (DIU); 3′-azido-2′,3′-dideoxyuridine (AZU or CS-87), 2′,3′-dideoxy-2′,3′-dihydrocytidine (DDC); 2′,3′-dideoxythymidine (DDT); 3′-fluoro-3′-deoxythymidine (FDT); 2′,3′-dideoxy-2′,3′-didehydrothymidine (D4T); 2′,3′-dideoxy-2′,3′-dehydroxytidine (D4C), [Clin. Prod. Rev. contents November 1987, 20 - 27; Trends in Pharmacol. Sci 8, 339-345 (1987); Chem. Eng. News November 23, 1987. 41-49; J. Med. Chem. 30, 1270-1278 (1987); ibid 31, 336-340 (1988)], Chu et al; U.S. Patent No. 4,681,933; Mitsuya et al, in AIDS, Broder, S. Ed., Marcel Dekker, Inc., N.Y., 1987, P. 303-332].

Examples of purine nucleosides having antiviral activity against the human immunodeficiency virus (HIV, synonymous with LAV or HTLV-III) include 2′,3′-dideoxy-2′,3′-dihydroadenosine (DDA); 3′-azido-2′3′-dideoxyadenosine (AZA); 2′,3′-dideoxy-2′,3′-didehydroadenosine (DDDA); 3′-azido-2′-3′-dideoxyguanosine (AZG); and others [Chem. Eng. News November 23, 1987, 41-49; Trends in Pharmacol. Sci. 8, 339-345 (1987); J. Med.Chem. 30, 2131-2137 (1987)].

The above described compounds have the formulae given herebelow.

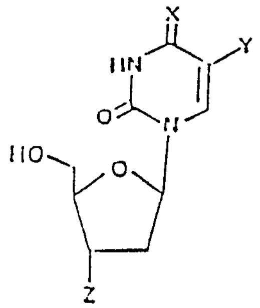

AZT: X=O, Y=$CH_3$, Z=$N_3$
AZU: X=O, Y=H, Z=$N_3$
DDC: X=NH, Y=H, Z=H
DDT: X=O, Y=$CH_3$, Z=H
DBU: X=O, Y=Br, Z=$N_3$
FDT: X=O, Y=$CH_3$, Z=F
DIU: X=O, Y=I, Z=$N_3$

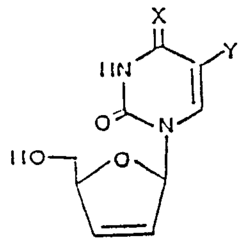

D4T: X=O, Y=$CH_3$
D4C: X=NH, Y=H

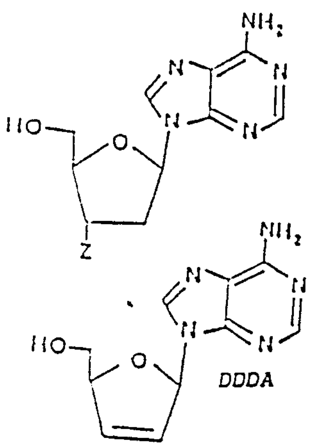

DDA: Z=H
AZA: Z=$N_3$

DDDA

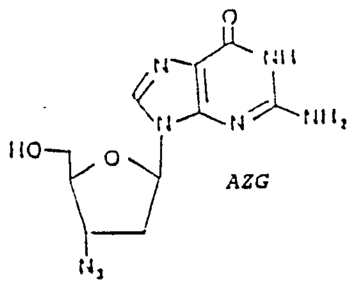

AZG

AZT (Retrovir[R]) is sequentially metabolized to the 5′-mono-, -di-,and-triphosphate analogues. As the triphosphate analogue, AZT inhibits the utilization of (dTTP) by reverse transcriptase and incorporated in the terminal position of DNA, thereby preventing elongation [Proc. Natl. Acad. Sci. USA 83, 8333 (1986)]. AIDS patients survive longer when taking AZT, show clinical improvements such as weight gains, have fewer bouts of opportunistic infections, and exhibit an increase in the T-helper cell number [Clin. Prod. Rev. Contents November 1987, 20-27; Chem. Eng. News November 1987, 41-49].

AZT is the drug which is currently advocated for the treatment of AIDS patients. Deleteriously, it suffers from a rather short half-life in the body [Lancet 557 (1986)] and furthermore suppresses bone marrow cell formation [Trends in Pharmacol. Sci. 8, 339-345 (1987)]. Therefore, there is an urgent need for other compounds that are equally potent but more selective in their antiviral (i.e. anti-AIDS) action.

2′,3′-Dideoxy-2′,3′-didehydro and 2′,3′-dideoxy-2′,3′-dihydrocytidine and adenosine derivatives are very active against HIV. However, these compounds suffer from the action of cytidine/deoxycytidine deaminase [J. Med. Chem. 30, 1270-1278 (1987); ibid 30, 440-444 (1987)] and adenosine deaminase [J. Med. Chem. 30, 2131-2137 (1987)] generating products that are inactive against HIV.

Ashwell et al., Nucl. Acids Res., 15, 2157-2166 (1987); Wu et al., Tetrahedron, 44, 6705-6722 (1988); Ueda, T., Japan Pat. No. J5 2027-782; Verheyden et al., U.S. Pat. No. 3,817,982; Horwitz et al., J. Org. Chem. 31, 205-211 (1966), have disclosed some 2′,3′-unsaturated-2′,3′-dideoxynucleosides pyrimidine derivatives. However, none of these compounds have the particular Z substituent disclosed in the present invention. Furthermore, the anti-HIV properties of 3′-substituted-2′,3′-dehydro-2′3′-dideoxy-nucleosides were not known heretofore.

In view of the prior art, and the lack of effective treatment for AIDS, it is clear that there remains a strong need for new antiviral agents having low toxicity to normal cells.

## SUMMARY OF THE INVENTION

The present invention is based on the discovery that certain 2′,3′-didehydro-2′,3′-dideoxy pyrimidine derivatives exhibit inhibitory activity towards viruses such as HIV in-vitro, yet at the same time exhibit low toxicity towards host cells (vero cells).

In accordance with the present invention, there is provided a 1-(3′-substituted-2′,3′-didehydro-2′,3′-dideoxyribofuranosyl) derivative of the general formula I:

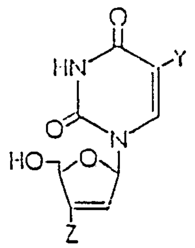

.....I

wherein Y is H, or a $C_1$-$C_6$ alkyl group; and
Z is an azido group, or a 1-(1,2,3-triazolo) group, or a cyano group, or an optionally substituted alkynyl group, or an optionally substituted alkenyl group, or an optionally substituted sulfide group or an optionally substituted sulfone group.

In accordance with another definition of the present invention there is provided a 1-(3′-substituted-2′,3′-didehydro-2′,3′-dideoxyribofuranosyl) derivative having the general formula

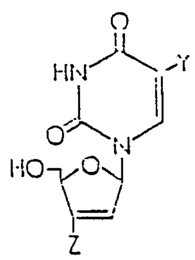

....I

wherein Y is H, or a $C_1$-$C_6$ alkyl group; and
Z is an azido group, or a 1-(1,2,3-triazolo group), or a cyano group, or an optionally substituted alkynyl group, or an optionally substituted alkenyl group, or an optionally substituted sulfide group or an optionally substituted sulfone group.

Preferably, the optional substituents for the alkynyl group and alkenyl group are selected from H, or a $C_1$-$C_6$ alkyl group or a phenyl group.

Preferably, the optional substituents for the sulfide group and sulfone group are selected from a cyano group, or a lower alkyl group or a phenyl group or a heterocyclic aromatic moiety. Preferably the lower alkyl group is selected from methyl, ethyl, propyl, isopropyl, cyclopropylmethyl, cyclopropyl, butyl, isobutyl, sec-butyl, tert-butyl, pentyl or hexyl. Preferably the heterocyclic aromatic moiety is selected from the group comprising: 2-pyrimidinyl, 2-pyridinyl, 2-(1-methyl)imidazolo, 5-(1-methyl) tetrazolo, 2-benzimidazolo, 2-benzoxazolo, 2-benzthiazolo, 2-(4-methyl)-1,3,4-thiadiazolo, 3-1H-1,2,4-triazolo, 4-2H-1,2,3-triazolo, 2-(4-methyl)thiazolo, 2-thiazolinyl).

A preferred group of compounds as those wherein Y is selected from H or $CH_3$, and Z is $SO_2R_2$ wherein $R_2$ is selected from the group of optional substituents for the sulfone group described supra.

Preferred compounds of this invention are as follows:
1-(3′-azido-2′,3′-didehydro-2′,3′-dideoxyribofuranosyl)thymine;
1-[2′,3′-didehydro-2′,3′-dideoxy-3′-(1″-1″,2″,3″-triazolo) ribofuranosyl]thymine;
**1-(2′,3′-didehydro-2′,3′-dideoxy-3′,** -ethynylribofuranosyl)thymine;
1-(2′,3′-didehydro-2′,3′-dideoxy-3′-(2″-sulfono-5″-methyl-1″,3″,4″-thiadiazolo) ribofuranosyl]thymine;
1-[2′,3′-didehydro-2′,3′-dideoxy-3′-(2″-sulfono-4″-methyl-thiazolo) ribofuranosyl]thymine;
1-(2′,3′-didehydro-2′,3′-didehydro-3′-cyanoribofuranosyl)thymine;
2′,3′-didehydro-2′,3′-dideoxy-3′-(2″-sulfono-5″-methyl)thiadiazolo) uridine
2′,3′-didehydro-2′,3′-dideoxy-3′-ethynyluridine; and
2′,3′-didehydro-2′,3′-dideoxy-3′-cyanouridine.

In its most preferred embodiment, the compound of Formula I comprises: 1-[2′,3′-didehydro-2′,3′-dideoxy-3′-(2″-sulfono-5″-methyl-1″,3″,4″-thiadiazolo)ribofuranosyl.

It has been determined that physiologically acceptable compounds of Formula I possess valuable pharmacological properties. More specifically, such compounds exhibit activity against HIV, HTLV-III, and LAV.

Thus the compounds of Formula I may be utilized as active compounds in medicaments, being formulated with a pharmaceutically acceptable carrier.

## DESCRIPTION OF THE DRAWINGS

Figure 1 is a schematic showing a general synthetic route for preparing the compounds of the present invention.

Figure 2 is a graph demonstrating the effect of 1-[2′,3′-didehydro-2′-3′-dideoxy-3′-(2″-sulfono-5″-methyl-1″,3″,4″-thiadiazole)ribofuranosyl]thymine Compound No. 5 on the in-vitro inhibition of the killing of HIV-infected T4 lymphocytes.

## DESCRIPTION OF THE PREFERRED EMBODIMENT

The compounds of formula I may be prepared using the general synthetic approach outlined in Figure 1. In general, the 1-(3′-2 substituted-2′,3′-didehydro-2′,3′-dideoxyribofuranosyl)pyrimidine derivatives of formula I are prepared as follows.

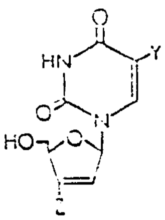

...I

The 1-(2′,3′-epoxy-5′-O-protected-β-D lyxofuranosyl) pyrimidine of formula II is first converted to the 1-(3′-Z-substituted-5′-O-protected-3′-deoxyarabinosyl)pyrimidine of formula III initially by nucleophilic addition on the 3′ position using a suitable synthetic pathway as will be detailed hereinafter. Z is an azido, or a 1-(1,2,3-triazolo group, or a cyano group, or a optionally substituted alkynyl group, or an optionally substituted alkenyl group, or an optionally substituted sulfide group or an optionally substituted sulfone group.

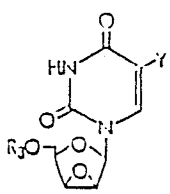

...II

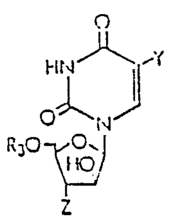

...III

$R_3$ represents the usual protecting groups in nucleoside chemistry, such as trityl, dimethoxytrityl, trimethylsilyl, t-butyldimethylsilyl, p-nitrobenzoyl, benzoyl, acetyl or the like. Y represents a hydrogen atom or lower alkyl moiety.

The 1-(3′-Z-substituted -5′-O-protected-3′-deoxyarabinosyl)pyrimidine derivatives of formula III is reacted with methanesulfonyl chloride using standard conditions employed in nucleoside chemistry to give a derivative having the general formula IV.

For example, the reaction may be carried out in pyridine at 0°C followed by 25°C for a period of 0.5 to 17 hours. Alternatively, the reaction may be carried out in methylene chloride containing triethylamine at 0°C followed by 25°C for a period of 0.5 to 17 hours.

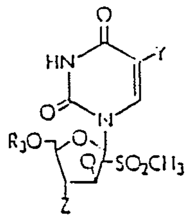

...IV

Reaction of compound IV with strong bases, provides the 1-(2′,3′-Z-substituted-didehydro-5′-O-protected-2′,3′-dideoxyribofuranosyl)pyrimidine derivatives of formula IV.

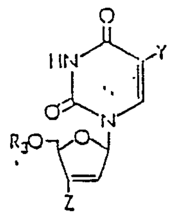

...V

Suitable bases can be selected from sodium hydroxide in absolute ethanol, sodium ethoxide in ethanol, sodium methoxide in methanol, or potassium t-butoxide in t-butanol.

Removal of the 5′-O-protecting group of compound V to provide compounds of the general formula I is carried out as follows. When $R_3$ is trityl or dimethoxytrityl group it can be removed by acid hydrolysis. For example 80% acetic acid at 20°C to about 100°C for 10 minutes to 17 hours. Alternatively 0.1 M to 1.0M hydrochloric acid in acetone, water or methanol, water or ethanol or water mixtures at about 20°C to 100°C for a period of 10 minutes to 17 hours. When $R_3$ is a trimethylsilyl or t-butyldimethylsilyl group, it is removed with tetrabutylammonium fluoride in tetrahydrofúran or dimethoxyethane at about 10°C to about 50°C for a period of 0.5 to 17 hours. When $R_3$ is a p-nitrobenzoyl group, it can be removed by treatment with sodium methoxide in dioxane-methanol mixtures at 0°C to 50°C for a period of 0.5 to 10 hours. When $R_3$ is an acetyl group, it can be removed by treatment with aqueous ammonia and methanol at about 0°C to about 40°C for a period of 0.5 to 48 hours.

More specifically, a method for the preparation of the 1-(3′-nitrogen substituted -2′,3′- didehydro-2′,3′-dideoxyribofuranosyl)pyrimidine derivatives wherein Z is an azido moiety is given as follows. Usable as azide anions are sodium azide, ammonium azide or the like. The reaction of compound of the formula (II), wherein $R_3$ is trityl and Y is a methyl group, with either previously mentioned azide reagents is best performed in an amount of about 1-10 moles of azide per mole of compound of the formula (II). The use of about 5 moles of azide per mole of compound of the formula (II) is preferred. Any polar aprotic solvent can be used, provided that they can dissolve the reagents. Thus, hexamethylphosphoric triamide, dioxane, acetonitrile, pyridine, dimethylformamide, dimethylsulffoxide, among others, are preferably used. The reaction is carried out generally at temperatures of about 25°C to about 150°C, preferably about 90°C to 110°C, and generally for a period of about 0.5 - 17 hours. The product from this reaction, 1-(3′-azido-5′-O-protected-3′-deoxyarabinosyl)pyrimidine is given below as a compound of the formula VI.

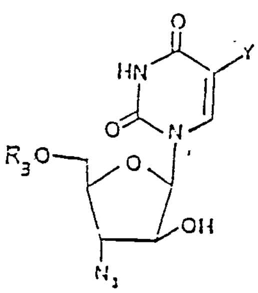

...VI

Compounds of the formula VI are converted to compounds of the formula I by the general synthetic pathway described supra.

Furthermore, reaction of the compounds of the formula VI with alkynes, such as acetylene, produce compounds of the formula VII as given below.

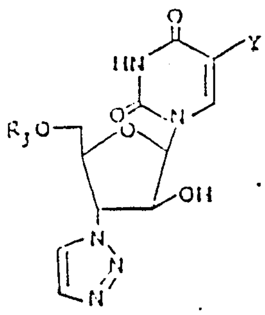

...VII

Using similar synthetic strategy to that described above, compounds of the formula VII are converted to compounds of the formula I.

The 1-(3′-alkynyl-2′,3′-dehydro-2′,3′-dideoxyribofuranosyl)pyrimidine derivatives of formula (I) according to the invention are produced, for example, by nucleophilic addition on the 3′ position of 1-(2′,3′-epoxy-5′-O-protected-β-D-lyxofuranosyl)pyrimidine of formula (II) with an alkynyl anion. Suitable alkynyl anions would include sodium acetylide, lithium acetylide, or the like.

The reaction of compounds of the formula II, wherein $R_3$ is trityl and Y is a methyl group, with either of the above-mentioned acetylide reagents is best performed in an amount of about 2-10 moles of acetylide per mole of compound of formula II. The use of about 2.7 moles of acetylide per mole of compound of formula II is preferred. Any polar aprotic solvents can be used, provided that they can dissolve the reagents. Thus, hexamethylphosphoric triamide, dimethylformamide, dimethylsufloxide, dioxane, pyridine, among others, are preferably used. The reaction is carried out generally at temperatures of about 15°C to about 30°C, preferably at 22°C and generally for a period of 96 to 144 hours. The product from this reaction is 1-(3′-deoxy-3′-ethynyl-5′-O-tritylarabinosyl)-pyrimidine having the formula VIII given below.

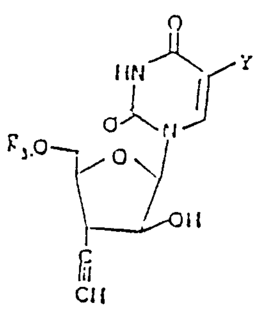

...VIII

Compounds of the formula VIII are converted to compounds of the formula I using the approach described earlier herein.

The 1-(3′-sulfur substituted-2′,3′-dehydro-2′,3′-dideoxyribofuranosyl)pyrimidine derivatives of formula (I) are produced, for example, by nucleophilic addition on the 3′ position of 1-(2′,3′-epoxy-5′-O-protected-β-D-lyxofuranosyl)pyrimidine of formula II with a mercapto functional group in the presence of a base.

For example reaction of mercapto compounds with 1-2′,3′-epoxy-5′-O-protected-β-D-lyxofuranosyl)thymine gave the 1-(3′-deoxy-3′-sulfur substituted-5′-O-tritylarabinosyl)pyrimidine of the formula IX. $R_3$ was trityl and Y was methyl. The reaction is performed in water:acetone, water:methanol, water:ethanol, and water:dimethoxyethane mixtures at 22°C to 100°C for 4 to 48 hours. The use of one to two moles of mercapto compound per mole of compound of the formula II is preferred for the reaction, although higher mole ratios are possible.

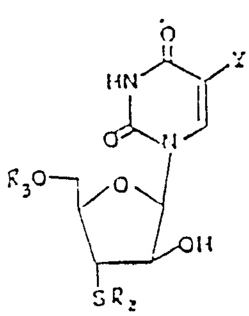

...IX

Oxidation of compound (IX) with metachloroperoxybenzoic acid in methylene at 0°C to 40°C for 0.5 to 18 hours gave the desired sulfone product of the formula X:

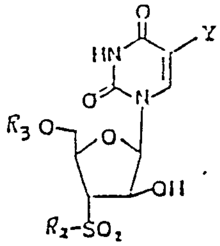

...X

wherein $R_3$ is trityl, Y is methyl and $R_2$ is one of the optional substituents as defined earlier. Other oxidizing reagents, such as peroxyacetic acid, sodium periodate, hydrogen peroxide and potassium permanganate can also be used for this reaction.

The compounds of formula I can be prepared from the compounds of formula IX or X as described supra.

The preparation of 1-(3′-cyano-2′,3′-didehydro-2′,3′-dideoxyribofuranosyl)thymine derivative is given as follows. Usable cyanide anions are tetraalkylammonium cyanide, ammonium cyanide, sodium cyanide, potassium cyanide, diethylammonium cyanide, trimethylsilylcyanide or the like. The reaction of compounds of formula II, wherein $R_3$ is t-butlydimethylsilyl and Y is a methyl group, with diethylaluminum cyanide is best performed in freshly dried and distilled solvents such as tetrahydrofuran chloroform, methylene chloride, acetonitrile. The reaction can be performed using 1 to 10 moles of diethylaluminum cyanide per mole of compound of formula II; however, the use of 4 moles is preferred. The reaction is carried out generally at temperatures of about 0-50°C; however, the use of temperature at 0°C followed by 25°C for a period of 1-48 hours.

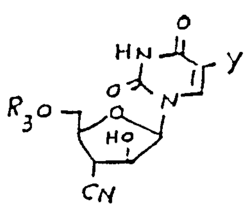

XI

Compounds of the formula XI are converted to compounds of the formula I by the general synthetic pathway described previously.

The compounds according to the invention as produced by the above method can be purified by means of separation such as recrystallization, column chromatography and reprecipitation.

The structures of the compound of the invention were established by the modes of synthesis, by infrared, by ultra violet, and by extensive high field (360 MHz) nuclear magnetic resonance (including two-dimensional methods, such as COSY) spectral techniques.

The compound of formula I, are useful as antiviral agents against the human immunodeficiency virus (HIV). Humans suffering from diseases caused by, for example, HIV can be treated by administering to the patient a pharmaceutically effective amount of one or more of the present compounds optionally, but preferably in the presence of a pharmaceutically acceptable carrier or diluent. There may also be included pharmaceutically compatible binding agent, and/or adjuvant materials. The active materials can also be mixed with other active materials which do not impair the desired action and/or supplement the desired action. The active materials according to the present invention can be administered by any route, for example, orally, parentally, intravenously, intradermally, subcutaneously, or topically, in liquid or solid form. For injection purposes, the medium used may be a sterile liquid. As an injection medium, it is preferred to use water which contains the stabilizing agents, solubilizing agents and/or buffers conventional in the case of injection solutions. Desirable additives include, for example, tartrate and borate buffers, ethanol, dimethylsulfoxide, complex forming agents (for example, ethylene diamine tetraacetic acid), high molecular weight polymers (for example, liquid polyethylene oxide) for viscosity regulation or polyethylene derivatives of sorbitan anhydrides.

Solid carrier materials include, for example, starch, lactose, mannitol, methyl cellulose, talc, highly dispersed silicic acid, high molecular weight fatty acids (such as stearic acid), gelatin, agar-agar, calcium phosphate, magnesium stearate, animal and vegetable fats or solid high molecular weight polymers (such as polyethylene glycols). Compositions suitable for oral administration can, if desired, contain flavoring and/or sweetened agents.

A preferred mode of administration of the compounds of this invention is oral. Accordingly, the compounds may be formulated into capsule form or tablet form.

The active materials according to the present invention can be employed in dosages and amounts which are conventional in the art. Thus, the materials can be used at a dosage range in humans of from 1 to 200 mg/kg

total body weight/day. A more preferred range lies between 1 to 30 mg/kg total body weight/day. A most preferred range is 5 to 20 mg/kg total body weight/day. The dosages may be administered at once, or may be divided into a number of smaller doses to be administered at varying intervals of time.

The in-vitro anti-HIV test results have shown that one of the present compounds in particular, 1-[2′,3′-didehydro-2′,3′-dideoxy-3′-(2″-sulfono-5″-methyl-1″,3″,4″-thiadazolo)ribofuranosyl]thymine (Compound No. 5) is active. The therapeutic index of a compound is determined by dividing the inhibitory or lethal concentration for 50% of the population ($IC_{50}$) by the effective concentration for 50% of the population ($EC_{50}$). The therapeutic index for 3′-azido-3′-deoxythymidine (AZT) and Compound No. 5 in cell culture is about 100 and 170, respectively.

As used in this invention, antiviral activity refers to the ability of a compound to inhibit the growth of a virus. The virus of primary importance with respect to the present invention is HIV. However, the present compounds may also exhibit antiviral activity towards other retroviruses. For example, AZT exhibits activity against murine retroviruses [J. Med. Chem. 30, 440-444 (1987)].

The ability of a compound to inhibit HIV may be measured by various experimental techniques. One such technique involves the inhibition of the killing of HIV-infected T4-lymphocytes.

## EXPERIMENTAL EXAMPLES

### Synthesis of Compounds

Some exemplary syntheses of specific compounds of this invention are as follows:

### Example 1

1-(2′,3′-Epoxy-5′-O-trityl-β-D-lyxofuranosyl)thymine
[Compound (II), $R_3$ is trityl, Y is methyl].

To a chilled (0°C) solution of 1-(2′,3′-O-dimethanesulfonyl-5′-O-tritylribofuranosyl)thymine (30.4 g), 46.3 mmole) in acetone (100 ml) was slowly added an aqueous solution of 2M sodium hydroxide (100 ml). The resulting reddish solution was stirred at about 22°C under an atmosphere of nitrogen for 20 hours. The solution was cooled in an ice-water bath, then slowly neutralized to pH 7 with 1M HCl. The acetone present in the reaction mixture was removed under reduced pressure, and the remaining precipitate and water mixture was extracted with methylene chloride (200 ml, two times). The combined methylene chloride extracts were evaporated, and the residue was dissolved in a small amount of ethanol and chilled in an ice-water bath, then water was added to crystallize the produce. The off-white crystals were collected and dried in vacuo at room temperature to give 20.5 g (92% yield) of 1-(2′,3′-epoxy-5′-O-trityl-β-D-lyxofuranosyl)thymine.

Melting point: 168°C - 170°C.

$^1$H-NMR (60 MHz, DMSO-$d_6$): δ 11.43 (s, NH3), 7.45 (s, br, 16H, $Ph_3C$ and H6), 6.17 (s, H1′), 4.26 (t, H4′), 4.09 (s, H2′, H3′), 3.30 (m, H5′), 1.67 (s, $CH_3$).

IR (Nujol): ν 3200, 1705, 1680, 1075 cm-1.

### EXAMPLE 2

1-(3′-Azido-3′-deoxy-5′-O-tritylarabinosyl)thymine
[Compound (III), $R_3$ is trityl, Y is methyl].

The solution of sodium azide (2.69 g, 41.4 mmole), 1-(2′,3′-epoxy-5′-O-trityl-β-D-lyxofuranosyl)thymine (4.00 g 8.29 mmole) in dry dimethylsulfoxide [the DMSO was dried over molecular sieves 3A°] (75 ml) was heated to about 110°C for 18 hours. The solvent was removed under reduced pressure, and the residue was taken up in ethyl acetate (400 ml). The ethyl acetate layer was washed with water (2 times 200 mL) then dried over anhydrous sodium sulfate. Removal of solvent gave a yellow foam which was dissolved in a small amount of methylene chloride and chilled to about 0°C, then precipitated by the addition of hexane. The white solid was collected and dried in vacuo to give 3.74 g of 1-(3′-azido-3′-deoxy-5′-tritylarabinosyl)thymine (86% yield).

$^1$H-NMR (MHz, DMSO-$d_6$): δ 11.40 (s, NH3), 7.40 (s br, $Ph_3C$, H6), 6.17 (d, 5.0, H1′, 2′-OH), 4.35 (m, H2′, H4′), 3.90 (m, H3′), 3.40 (m, H5′), 1.67 (s, $CH_3$).

IR (Nujol): ν 3325, 2100, 1700, 1670, 1075, 705 cm-1.

### EXAMPLE 3

1-(3′-Azido-3′-deoxy-2′-O-methanesulfonyl-5′-O-tritylarabinosyl)thymine
[Compound (IV), $R_3$ is trityl, Y is methyl].

To a chilled (0°C) solution of 1-(3′-azido-3′-deoxy-5′-O-tritylarabinosyl)thymine (2.10 g, 4.00 mmole) in dry methylene chloride (dried over molecular sieves 3A°, 60 mL) containing dry triethylamine (dried over molecular sieves 3A°), 0.79 mL, 5.60 mmole) was added dropwise a solution of methanesulfonyl chloride (0.37 mL, 4.80 mmole) in dry methylene chloride (20 mL). The resulting solution was stirred at about 22°C for 18 hours, then poured into ice-water (200 mL), and extracted with methylene chloride (2 times 100 mL). The combined organic extracts were washed successively with water (once, 50 mL), 5% sodium bicarbonate (once, 50 mL), water (once, 50 mL) then dried over anhydrous sodium sulfate. Removal of the solvent gave an off-white foam of 1-(3′-azido-3′-deoxy-2′-O-methanesulfonyl-5′-O-tritylarabinosyl)thymine (2.36 g, 98% yield), which was homogeneous by TLC analysis.

$^1$H-NMR (60 MHz $CDCl_3$): δ 9.17 (s br, NH3), 7.40 (s br, $Ph_3C$, H6), 6.25 (d, 6.0, H1′), 5.25 (t, 6.0, H2′), 4.45 (m, H4′), 3.85 (m, H3′), 3.50 (m, H5′), 2.90 (s, $SO_2CH_3$), 1.70 (s, $CH_3$).

## EXAMPLE 4

1-(3′-Azido-2′,3′-didehydro-2′,3′-dideoxyribofuranosyl)thymine
[Compound (I), Y is methyl, Z is azido].

1-(3′-azido-3′-deoxy-2′-O-methanesulfonyl-5′-O-tritylarabinosyl)thymine (1.21 g, 2.00 mmole) was added to a freshly prepared solution of 1M sodium ethoxide in absolute ethanol (50 mL). The suspension was heated under reflux for 1 hour under nitrogen atmosphere, at which time a reddish solution was formed and thin layer chromatography showed no starting material left. The reaction mixture was chilled in an ice-water bath, then it was acidified to pH 3 with 6M hydrochloric acid. Ice-water (200 mL) was added to the acidic reaction mixture, and the resulting precipitate was collected. Thin layer chromotography analysis of the precipitate (silica gel, 10% methanol in chloroform) showed the presence of the hydrolysis product 1-(3′-azido-3′-deoxy-5′-O-tritylarabinosyl)thymine and the desired elimination product 1-(3′-azido-2,3′-dehydro-2′,3′-dideoxy-5′-O-tritylribofuranosyl)thymine [Compound (V), $R_3$ is trityl, Y is methyl]. The precipitate containing the mixture of hydrolysis and elimination products was used directly in the next detritylation step.

The above precipitate was dissolved in 80% acetic acid (40 mL) and heated to about 80°C for 0.5 hour. Water (200 mL) was added, and the precipitated triphenylmethanol was removed. Concentration of the filtrate gave a brown foam which was coevaporated with methanol (two times, 50 mL). The residue was purified by column chromotography (silica gel, 2.5% methanol in chloroform) to give the desired less polar elimination product 1-(3′-azido-2′,3′-dehydro-2′,3′-dideoxyribofuranosyl)thymine (30 mg, 5% yield) as an off-white solid. Recrystallization of this material from 95% ethanol gave 27 mg of white crystals, which was homogeneous by TLC analysis.

Melting point: 145°C (decomposition)
$^1$H-NMR spectrum (Bruker WM-360, 360 MHz, DMSO-$d_6$): $^1$-NMR δ 11.44 (s, NH3), 7.99 (d, 1.2, H6), 6.73 (dd, 1.7, 3.4, H1′), 6.07 (t, 1.7, H2′), 5.10 (s br, 5′ - OH), 4.84 (dd, 2.0, 3.4, H4′), 3.59 (s br, H5′), 1.76 (d, 1.2, $CH_3$).
IR(Nujol): ν 3500, 3188, 3075, 2125, 1700, 1660 $cm^{-1}$.
UV (95% ethanol): λ max 255 mm.

The more polar hydrolysis product 1-(3′-azido-3′-deoxyarabinosyl)thymine (510 mg, 72% yield) was obtained as a white powder.

Melting point: 171°C - 172°C (recrystallized from 95% ethanol).
$^1$H-NMR (360 MHz, DMSO-$d_6$): δ 11.29 (s, NH3), 7.60 (s, H6), 6.09 (d, 6.5, 2′-OH), 6.03 (d, 6.5, H′), 5.34 (t, 4.9, 5′-OH), 4.36 (q, 6.5, H2′), 4.05 (t, 6.5, H3′), 3.65 (m, H5′), 1.77 (s, $CH_3$).
IR(Nujol): ν 3350, 3180, 2110, 1700, 1660 $cm^{-1}$.

## EXAMPLE 5

1-[3′Deoxy-3′-(1″-1″,2″,3″-triazolo)-5′-O-tritylarabinosyl]thymine
[Compound (VI), $R_3$ is trityl, Y is methyl].

A chilled (-78°C) solution of 1-(3′-azido-3′-deoxy-5′-O-tritylarabinosyl)thymine (0.53 g, 1.0 mmole) in dry acetone (dried over molecular sieves 3A°, 40 mL) in a steel bomb was condensed in acetylene/acetone (1.20 g). The sealed steel bomb was heated to about 85°C for 19 hours. The bomb was cooled to ambient temperature, and the excess acetylene removed. Concentration of the reaction mixture gave a foam which was dissolved in a small amount of methylene chloride, and chilled (0°C), then precipitated with hexane to give a white solid. The solid was further purified by medium pressure liquid chromatography (silica gel, 10% methanol in chloroform) to give 0.50 g (90% yield) of 1-[3′-deoxy-3′-(1″-1″,2″,3″-triazolo)-5′-O-tritylarabinosyl]thymine as a white powder.

$^1$H-NMR (60 MHz, DMSO-$d_6$): δ 9.489 (s br, NH3), 8.29 (s, triazole 1H), 7.85 (s, triazole 1H), 7.40 (s br,

$Ph_3C$,H6), 6.40 (d, 6.0, H1′), 6.22 (d, 6.0, 2′-OH), 5.35 (m, H4′), 4.83 (q, 6.0, H2′), 4.49 (m, H3′), 3.36 (m, H5′), 1.67 (s, $CH_3$).
IR (Nujol: ν 3300, 3150, 1690, 1665, 1280, 1070, 705 $cm^{-1}$.

EXAMPLE 6

1-(3′-Deoxy-2′-O-methanesulfonyl-3′-(1″-1″,2″,3″-triazolo)-5′-O-tritylarabinosyl]thymine

To a chilled (0°C) solution of 1-[3′-deoxy-3-(1″-1″,2″,3″-triazolo)-5′-O-tritylarabinosyl]thymine (2.10g, 3.81 mmole) in dry methylene chloride (10 mL) containing dry triethylamine (0.74 mL, 5.33 mmole) was added dropwise a solution of methansulfonyl chloride (0.35 mL, 4.57 mmole) in dry methylene chloride (5 mL). The resulting reaction mixture was stirred at 22°C for 1.5 hour, then poured into ice-water (100 mL) and extracted with methylene chloride (twice, 100 mL each). The combined methylene chloride extracts were washed successively with water (50 mL), 5% sodium bicarbonate (50 mL), water (50 mL), then dried over anhydrous sodium sulfate. Removal of the solvent gave an off-white foam of 1-[3′-deoxy-2′-O-methanesulfonyl--3′-(1″-1″,2″,3″-triazole)-5′-O-tritylarabinosyl]thymine (2.35 g, 98% yield), which was homogeneous by TLC analysis, and used directly in the next reaction.

EXAMPLE 7

1-[2′,3′-Didehydro-2′,3′-dideoxy-3′-(1″-1″,2″,3″-triazolo)ribofuranosyl]thymine
[Compound (I), Y is methyl, Z is a 1″-1″,2″,3″-triazolo group].

1-[3′-Deoxy-2′-O-methanesulfonyl-3′-(1″-1″,2″,3″-triazolo)-5′-O-tritylarabinosyl]thymine (2.34 g, 3.72 mmole) was added to a freshly prepared solution of 1M sodium hydroxide in a 1:1 ethanol:water mixture (50 mL). The suspension was heated to reflux for 5 hours, at which time a reddish solution was formed. The reaction mixture was chilled in an ice-water bath then acidified to pH 3 using 6M hydrochloric acid. Ice-water (100 mL) was added to the acidic reaction mixture, and the resulting precipitate was collected and dried in vacuo to give 1.78g (90% yield) of 1-[2′,3′-dehydro-2′,3′-dideoxy-3′-(1″-1″,2″,3″-triazolo)-5′-O-tritylribofuranosyl]thymine as a white powder which was used directly in the next detritylation step.

The above white powder was dissolved in 80% acetic acid (40 mL) and heated to about 80°C for 5.5 hours. Water (200 mL) was added, and the precipitated triphenylmethanol was removed. Concentration of the filtrate gave an oily residue which was coevaporated with methanol (three times, 50 mL each) to give a solid residue. The solid was recrystallized from 95% ethanol to give 1-[2′,3′-Dehydro-2′,3′-dideoxy-3′-(1″-1″,2″,3″-triazolo)ribofuranosyl]thymine (0.43 g, 39% yield) as white crystals, which was homogeneous by TLC analysis.
Melting point: 186°C (decomposition).
$^1$H-NMR (360 MHz, DMSO-$d_6$): δ 11.43 (s,NH3), 8.96 (s, triazole 1H), 7.98 (s, triazole 1H), 7.00 (s, H1′), 6.49 (s,H2′), 5.44 (s, H4′), 5.22 (t, 1.8, 5′-OH), 4.05 (d, 7.4, H5′), 3.77 (dd, 1.8, 7.4, H5″), 1.71 (s, $CH_3$).
IR (Nujol): ν 3313, 3188, 3050, 1688, 1459 $cm^{-1}$.
UV (95% ethanol): λ max 251 mm.

EXAMPLE 8

1-(3′-Deoxy-3′-ethynyl-5′-O-tritylarabinosyl)thymine
[Compound (VIII), $R_3$ is trityl, Y is methyl].

A solution of lithium acetylide ethylenediamine complex (2.06 g, 22.4 mmole) in dry dimethylsulfoxide (dried over molecular sieves 3A, 30 mL) was stirred at 22°C for 1 hour, then slowly added to a solution of 1-(2′,3′-epoxy-5′-O-trityl-β-D-lyxofuranosyl)thymine (4.00 g, 8.28 mmole) in dry dimethylsulfoxide (5 mL). The resulting brown solution was stirred at 22° for 120 hours. The solvent was removed under reduced pressure, and the residue was taken up in ethyl acetate (400 mL). The ethyl acetate extract was washed with water (twice, 200 mL each) then dried over anhydrous sodium sulfate. Removal of the solvent gave a brown foam which was purified by column chromotography (silica gel, 5% methanol in chloroform) to give a foam which was dissolved in a small amount of methylene chloride and chilled (0°C), then precipitated with hexane. The white powder was collected and dried in vacuo to give 3.72 g (90% yield) of 1-(3′-deoxy-3′-ethynyl-5′-O-tritylarabinosyl)thymine.
$^1$H-NMR (60 MNz, DMSO-$d_6$): δ 11.30 (s, NH3), 7.40 (s br, $Ph_3C$, H), 6.12 (d, 6.0, H1′), 6.00 (d, 6.0, 2′-OH), 4.45 (q, 5.0, H2′; t on $D_2O$ shake), 4.00 (m, H4′), 3.32 (m, H5′), 3.20 (m, H3′), 3.15 (d, 1.0, C=CH), 1.60 (s, $CH_3$).
IR (Nujol): ν 3300, 2120, 1700 (br), 1275, 1075, 760, 705 $cm^{-1}$.

## EXAMPLE 9

1-(3′-Deoxy-3′-ethynyl-2′-O-methanesulfonyl-5′-O-tritylarabinosyl)thymine
$R_3$ is trityl, Y is a methyl group].

To a chilled (0°C) solution of 1-(3′-deoxy-3′-ethynyl-5′-O-tritylarabinosyl]thymine (2.40 g, 4.83 mmole) in dry methylene chloride (dried over molecular sieves 3A, 60 mL) and dry triethylamine (dried over molecular sieves 3A, 0.94 mL, 6.76 mmole) was added dropwise a solution of methane-sulfonyl chloride (0.45 mL, 580 mmole) in dry methylene chloride (10 mL). The resulting solution was stirred at 22°C for 1 hour, the poured into ice-water (100 mL) and extracted with methylene chloride (2 times, 100 mL each). The combined methylene chloride extracts were washed successively with water (50 mL), 5% aqueous sodium bicarbonate (50 mL), water (50 mL), then dried over anhydrous sodium sulfate. Removal of the solvent gave a tan foam of 1-(3′-deoxy-3′-ethynyl-2′-O-methanesulfonyl-5′-O-tritylarabinosyl)thymine (2.55 g, 92% yield) which was homogeneous by TLC analysis.

$^1$H-NMR (60 MHz, $CDCl_3$): δ 9.80 (s, NH3), 7.40 (s br, $Ph_3C$, $H_6$), 6.30 (d, 6.0, H1′), 5.37 (t, 6.0, H2′), 4.05 (m, H4′), 3.60 (m, H3′), 3.50 (m, H5′), 3.00 (s, $SO_2CH_3$), 2.35 (d, 1.0, C=CH).

## EXAMPLE 10

1-(2′,3′-Didehydro-2′,3′-dideoxy-3′-ethynylribofuranosyl)thymine
[Compound (I), Y is methyl and Z is ethynyl].

A clear yellow solution of 1-(3′-deoxy-3′-ethynyl-2′-O-methanesulfonyl-5′-O-tritylarabinosyl)thymine (1.27 g, 2.21 mmole) and 1M sodium hydroxide in a 1:1 ethanol:water mixture (40 mL) was heated to reflux, under an atmosphere of nitrogen, for 2 hours. The reaction mixture was chilled in an ice-water bath then acidified to pH 3 with 6M hydrochloric acid. Ice-water (200 mL) was added, and the resulting precipitate was collected and dried in vacuo to give 1-(2′,3′-dehydro-2′,3′-dideoxy-3′-ethynyl-5′-O-trityl-ribofuranosyl)thymine (0.98 g, 92% yield) as an off-white powder, which was used directly in the next detritylation step.

The above off-white powder was dissolved in 80% acetic acid (40 mL) was heated to about 80°C for 1.5 hour. On cooling, the crystallized triphenylmethanol was removed, and concentration of the filtrate gave an oily residue which was coevaporated with methanol (three times, 50 mL each) to give a tan foam. This foam was purified by column chromotography (silica gel, 5% methanol in chloroform) to give 1-(2′,3′-dehydro-2′,3′-dideoxy-3′-ethynylribofuranosyl)thymine (460 mg, 90% yield) as white crystals, which was homogeneous by TLC analysis.

Melting point: 125°C (decomposition).

$^1$H-NMR (360 MHz, DMSO-d6: δ 11.35 (s, NH3), 7.72 (d, 1.2, H6), 6.89 (dd, 1.4, 3,3, H1′), 6.29 (t, 1.5, H2′), 5.17 (t, 4.9, 5′-OH, disappear on $D_2O$ shake), 4.73 (dd, 2.0, 3.1, H4′), 4.56 (s, C≡CH), 3.68 (dd, 2.5, 4.7, H5′), 1.73 (s, $CH_3$).

IR (Nujol): ν 3438, 3124, 2109, 1694, 1656, 1266, 1175, 1113, 1056, 963, 843, 768, 588 $cm^{-1}$.

′V (95% ethanol): λ max 265 nm.

## EXAMPLE 11

1-[3′-Deoxy-3′-(2″-mercapto-5″-methyl-1″,3″,4″-thiadiazolo)-1′-O-tritylarabinosyl]thymine
[Compound (IX), $R_3$ is trityl, Y is methyl and $R_2$ is 2-mercapto-5-methyl-1,3,4-thiadiazolo].

A solution of 1-(2′,3′-epoxy-5′-O-trityl-β-D-lyxofuranosyl)thymine (4.00 g, 8.29 mmole), 2-mercapto-5-methyl-1,3,4-thiadiazole (1.64 g, 12.4 mmole) and sodium bicarbonate (1.18 g, 14.08 mmole) in acetone (75 mL) and water (50 mL) was heated under reflux for 17 hours. Concentration of the reaction mixture gave a solid residue which was dissolved in ethyl acetate (200 mL) and water (100 mL). The separated water layer was extracted with ethyl acetate (twice, 50 mL each). The ethyl acetate extracts were washed with 5% sodium hydroxide (twice, 50 mL each), water (once, 100 mL) and dried over anhydrous sodium sulfate. Removal of the solvent gave a tan foam which was purified by column chromotography (silica gel, 2.5% methanol in chloroform) to give 1-[3′-deoxy-3′-(2″-mercapto-5″-methyl-1″,3″,4″-thiadazolo)-5′-O-tritylarabinosyl]thymine as an off-white foam (3.17 g, 63% yield).

$^1$H-NMR (60 MHz DMSO-$d_6$): δ 11.40 (s, NH3), 7.40 (m, $Ph_3C$, H6), 6.35 (d, 5.0, H1′), 5.35 (d, 3.0, 2′-OH), 4.95 (m,H2′), 4.25 (s br, H3′, H4′), 3.62 (s, br, H5′), 2.68 (s, $CH_3$-thiadiazole), 1.68 (s, $CH_3$).

IR (Nujol): ν 3300, 1695 (br), 1265, 1075, 900, 700, 630 $cm^{-1}$.

## EXAMPLE 12

1-[3′-Deoxy-3′-(2″-sulfono-5″-methyl-1″,3″,4″-thiadiazolo)-5′-O-tritylarabinosyl]thymine

[Compound (X), $R_3$ is trityl, Y is methyl, $R_2$ is 2-mercapto-5-methyl-1,3,4-thiadiazolo].

To a chilled (0°C) stirring yellow solution of 1-[3′-deoxy-3′-(2″-mercapto-5″-methyl-1″,3″,4″-thiadiazolo)-5′-O-tritylarabinosyl]thymine (4.30 g, 7.00 mmole) in dry methylene chloride (180 mL) was added slowly metachloroperoxybenzoic acid (3.80 g, 80% pure, equivalent to 15.7 mmole). The resulting colorless clear solution was stirred at 22°C for 20 hours. The reaction mixture was diluted with $CH_2Cl_2$ (200 mL), then washed with water (twice, 50 mL), saturated sodium carbonate (once, 50 mL), water (once, 50 mL) and dried over anhydrous sodium sulfate. Removal of the solvent gave 1-[3′-deoxy-3′-(2″-sulfono-5″-methyl-1″,3″,4″-thiadiazolo)-5′-O-tritylarabinosyl]thymine (3.94 g, 88% yield) as a white foam.

$^1$H-NMR (60 MHz, DMSO-$d_6$): δ 11.55 (s, NH3), 7.40 (s br, $Ph_3C$, H6, 6.15 (d, 4.0, H1′, 2′-OH), 4.95 (m, H2′), 4.60 (m, 1H), 4.20 (m, 1H), 3.38 (m, H5′), 2.95 (s, $CH_3$-thiadiazole), 1.69 (s, $CH_3$).

IR (Nujol): ν 3300, 3200, 1695, 1340, 1155, 1125, 1075, 900, 700, 630, 600 $cm^{-1}$.

## EXAMPLE 13

1-[3′-Deoxy-2′-O-methanesulfonyl-3′-(2″-sulfono-5″-methyl-1″,3″,4″-thiadiazolo-5′-O-tritylarabinosyl-]thymine

$R_3$ is trityl, Y is methyl, $R_2$ is 2-mercapto-5-methyl-1,3,4-thiadiazolo].

To a chilled (0°C) stirring solution of 1-[3′-deoxy-3′-(2″-sulfono-5″-methyl-1″,3″,4″-thiadiazolo)-5′-O-tritylarabinosyl]thymine (2.49 g, 3.77 mmole) in dry methylene chloride (80 mL) and dry triethylamine (0.57 g, 5.66 mmole) was added dropwise a solution of methanesulfonyl chloride (0.38 mL), 4.90 mmole) in dry $CH_2Cl_2$ (20 mL). The resulting solution was stirred for 92 hours at 22°C. The solution was diluted with $CH_2Cl_2$ (100 mL), then washed with saturated sodium bicarbonate (once, 50 mL), water (once, 100 mL) then dried over anhydrous sodium sulfate. Removal of the solvent gave 1-[3′-deoxy-2′-O-methanesulfonyl-3′-(2″-sulfono-5″-methyl-1″,3″,4″-thiadiazolo)-5′-O-tritylarabinosyl]thymine (2.59 g, 91% yield) as a tan foam, which was used directly in the next reaction.

## EXAMPLE 14

1-[2′,3′-Didehydro-2′,3′-dideoxy-3′-(2″-sulfono-5″-methyl-1″,3″,4″-thiadiazolo)ribofuranosyl]thymine

[Compound (I), Y is methyl, $R_2$ is 2-sulfono-5-methyl-1,3,4-thiadiazolo].

To a solution of 1-[3′-deoxy-2′-O-methanesulfonyl-3′-(2″-sulfono-5″-methyl-1″,3″,4″-thiadiazolo)-5′-O-tritylarabinosyl]thymine (2.59 g, 3.45 mmole) in water:acetone (1:3, 30 mL) was added 1M hydrochloric acid (2.3 mL). The reaction mixture was heated to 80°C for 3 hours then chilled (0°C) and ice-water (200 mL) was added. The precipitated triphenylmethanol was removed. Concentration of the filtrate gave a residue which was purified by column chromotography (silica gel, 5% methanol in chloroform) to give 1-[2′,3′-dehydro-2′,3′-dideoxy-3′-(2″-sulfono-5″-methyl-1″,3″,4″-thiadiazolo) ribofuranosyl]thymine (656 mg, 66% yield) as a white solid.

Melting point: 116° (decomposition).

$^1$H-NMR (360 MHz, DMSO-$d_6$): δ 11.47 (s, NH3), 7.60 (s H6), 7.42 (s, H2′), 6.98 (d 2.0, H1′), 5.12 (m, 5′-OH, H4′), 3.72 (m, H5′), 2.91 (s, $CH_3$-thiadiazole), 1.74 (s, $CH_3$).

IR (Nujol): ν 3377, 3185, 1690, 1265, 1163, 1100 $cm^{-1}$.

UV (45% methanol:water): λ max 257 nm.

## EXAMPLE 15

1-[2′,3′-Didehydro-2′,3′-dideoxy-3′-(2″-sulfono-4″-methylthiazolo)ribofuranosyl]thymine

[Compound (I), Y is methyl, Z is 2-sulfono-4-methylthiazolo].

To a solution of 1-[3′-deoxy-2′-O-methanesulfonyl-3′-(2″-sulfono-4″-methylthiazolo)-5′-O-tritylarabinosyl-]thymine (2.75 g, 3.80 mmole) in acetone:water (3:1, 30 mL) was added 1M hydrochloric acid (2.3 mL). The reaction mixture was heated to 80°C for 4 hours then chilled (0°C) and ice-water (100 mL) was added. The precipitated triphenylmethanol was removed and the residue was washed with a methanol:water (1:1, 50 mL). Concentration of the filtrate gave a residue which was purified by column chromotography (silica gel, 2.5% methanol in chloroform) to provide 1-[2′,3′-dehydro-2′,3′-dideoxy-3′-(2″-sulfono-4″-methyl-thiazolo)ribofuranosyl]thymine (1.19 g, 81% yield) as a white solid.

Melting point: 109°C (decomposition).

$^1$H-NMR (360 MHz, DMSO-$d_6$): δ 11.44 (s, NH3), 7.99 (s H5-thiazole), 7.57 (s, H6), 7.24 (s, H2′), 6.95 (d, 2.0, H1′), 5.08 (t, 4.0, 5′-OH), 5.02 (m, H4′), 3.73 (dq, 2.0, 14.0, H5′), 2.49 (s, $CH_3$-thiazole), 1.72 (s, $CH_3$).

IR (Nujol): ν 3469, 3185, 3060, 1700 (br), 1331, 1263, 1160, 1150, 1095, 1063, 1043, 915, 865, 756, 621 $cm^{-1}$.

UV (45% methanol:water): $\underline{\lambda}$ max 278 nm.

EXAMPLE 16

1-(3′-Cyano-2″,3″-didehydro-2′,3′-dideoxyribofuranosyl)thymine
[Compound (I), Y is methyl and Z is cyano].

To a solution of 1-(3′-cyano-2′,3′-dehydro-2′,3′-dideoxy-tritylribofuranosyl)thymine (900 mg, 2.45 mmole) in dry THF (20 ml) was added a 1M solution of tetrabutylammonium fluoride in THF (3.71 ml, 3.71 mmole). After stirring for 1 hour at room temperature, the reaction mixture was concentrated under reduced pressure. The residue was purified by column chromotography (silica gel, 4% methanol in chloroform) to give 1-(3′-cyano-2′,3′-dehydro-2′,3′-dideoxyribofuranosyl)thymine (302 mg, 49% yield) as an off-white foam.

Melting point: 186°C-188°C

$^1$H-NMR (360 MHz, DMSO-$d_6$): $\underline{\delta}$ 11.43 (s, NH3, disappears with $D_2O$), 7.64 (s, TH6), 7.13 (s, H2′), 6.98 (d, 3.7, H1′), 5.40 (t, 4.4, 5′-OH, disappears with $D_2O$), 3.71 (s, H5′ and H5″), 1.73 (s, $TCH_3$).

IR (Nujol): $\underline{\nu}$ 3400, 3150, 2236, 1710, 1660 cm$^{-1}$.

UV (Ethanol): $\underline{\lambda}$ max 263 nm.

MS (FAB, glycerol): m/z 250 (M+1, 1%), 249 ($M^+$, 0.2%), 187 (M+1-2$H_2O$-HCN, 25%), 186 (M-2$H_2O$-HCN, 100%).

EXAMPLE 17

3′-Cyano-2′,3′-didehydro-2′,3′-dideoxyuridine
[Compound (I), Y is H and Z is CN].

$^1$H-NMR (60 MHz, DMSO-$D_6$): $\underline{\delta}$ 11.55 (s, $NH_3$, disappears with $D_2O$), 7.85 (d, 8.0, UH6), 7.20 (s, br, H2′), 7.08 (m, H1′), 5.70 (d, 8.0, UH5), 5.40 (t, 4.0, 5′-OH, disappear with $D_2O$), 3.80 (m, H5′, H5″).

IR (Nujol): $\underline{\nu}$ 3400, 3180, 2230, 1710, 1670 cm$^{-1}$.

EXAMPLE 18

2′,3′-Didehydro-2′,3′-dideoxy-3′-(2″-sulfono-5″-methyl-1″,3″-4″-thiadiazolo)uridine
[Compound (I), Y is H, Z is 2-sulfono-5-methyl-1,3,4-thiadiazolo].

$^1$H-NMR (60 MHz, DMSO-$d_6$): $\underline{\delta}$11.10 (s, br, NH3, exchanges with $D_2O$), 7.72 (d, 8.0, UH6), 7.45 (m, H2′), 7.05 (m, H1′), 5.68 (d, 8.0, UH5), 5.15 (m, H4′, 3′-OH which disappears with $D_2O$), 3.80 (m, H5′, H-5″) 2.90 (s, $CH_3$, thiadiazole).

IR (Nujol): $\underline{\nu}$ 3400-3200 (br), 2920, 2860, 1720, 1690 cm$^{-1}$.

EXAMPLE 19

2′,3′-Didehydro-2′,3′-dideoxy-3′-ethynyluridine
[Compound (I), Y is H, Z is C≡CH].

$^1$H-NMR (60 MHz, DMSO-$d_6$): $\underline{\delta}$ 11.49 (S, NH3, disappears with $D_2O$), 7.53 (d, 8.0, UH6) 6.95 (m, H1′), 6.45 (m, H2′), 5.74 (d, 8.0, UH5) 4.05 (m, H4′), 4.63 (s, -C=CH), 4.27 (m, H5′)

IR (Nujol): $\underline{\nu}$ 3400, 3200, 2104, 1700 (br) cm$^{-1}$.

EXAMPLE 20

1-(5′-t-Butyldimethylsilyl-3′-cyano-3′-deoxyarabinosyl)thymine
[Compound XI; Y is methyl, Z is cyano].

To a chilled (0°C) and stirred solution of 1-(t-butyldimethylsilyl-2′,3′-epoxylyxofuranosyl)thymine (3.00 g, 8.45 mmole) in dry THF (45 ml) was added dropwise a solution of 1M diethylammonium cyanide in toluene (33.9 ml, 33.9 mmole). After 30 minutes, the reaction was left stirring at room temperature for 22 hours. The reaction was quenched by the addition of 5% $NaHCO_3$ (100 ml), and the product was extracted with ethyl acetate (200 ml). The ethyl acetate extract was washed with water (once) then dried over anhydrous $Na_2SO_4$. Removal of the solvent gave a foam which was purified by column chromatography (silica gel, 3% methanol:97% $CHCl_3$) to give the desired product (1.75 g, 54% yield) as a white foam.

NMR $CDCl_3$: 10.65 (s,1H), 7.45 (s,1H), 6.25 (d br,4,1H), 5.15 (s br, 1H), 3.95-4.40 (m,2H), 3.35 (m,1H), 1.80 (s,3H).
IR (Nujol): $\underline{\nu}$ 2248, 1700 $cm^{-1}$.

## BIOLOGICAL DATA

### Antiviral Assay

The procedure used in the test for agents active against (HIV) is designed to detect agents acting at any stage of the virus reproductive cycle. The assay basically involves the killing of T4 lymphocytes by HIV. Small amounts of HIV are added to cells, and at least two complete cycles of virus reproduction are necessary to obtain the required cell killing. Agents which interact with virions, cells, or virus gene-products to interfere with viral activities will protect cells from cytolysis. The system is automated in several features to accommodate large numbers of candidate agents, and is generally designed to detect anti-HIV activity. However, compounds which degenerate or are rapidly metabolized in the culture conditions may not show activity in this screen. Also, while every attempt has been made to reduce variability, precise values for $EC_{50}$, $IC_{50}$, and Therapeautic Index are not possible. All tests are compared with a positive (AZT-treated) control done at the same time under identical conditions.

The Procedure:

1. T4 lymphocytes (CEM cell line) are exposed to HIV at a virus to cell ratio approximately 0.05, and plated along with noninfected control cells in 96-well microtiter plates.
2. The candidate agent is dissolved in dimethyl sulfoxide (unless otherwise instructed), then diluted 1:200 in cell culture medium. Further dilutions (half-$log_{10}$) are prepared before adding to an equal volume of medium containing either infected or noninfected cells.
3. Cultures are incubated at 37°C in a 5% carbon dioxide atmosphere for 6 or 7 days.
4. The tetrazolium salt, XTT, is added to all wells, and cultures are incubated to allow formazan color development by viable cells.
5. Individual wells are analyzed spectrophotometrically to quantitate formazan production, and in addition are viewed microscopically for detection of viable cells and confirmation of protective activity.
6. Drug-treated virus-infected cells are compared with drug-treated noninfected cells and with other appropriate controls (untreated infected and untreated noninfected cells, drug-containing wells without cells, etc.) on the same plate.
7. Data are reviewed in comparison with other tests done at the same time, and a determination about activity is made.

Using the above mentioned assay, the results are shown in Table I.

TABLE I

*Inhibition of the killing of HIV-infected T4-lymphocytes*

| Compound | $EC_{50}$ (ug/mL) |
|---|---|
| 1 | >100 |
| 2 | >100 |
| 3 | >100 |
| 4 | >100 |
| 5 | 4.9 |

a)

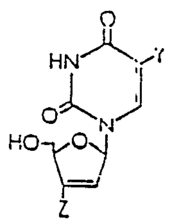

(I)

*Compound No. 1; wherein $Y=CH_3$, Z=azide in the general formula I;*

*Compound No. 2; wherein $Y=CH_3$, Z=triazole in the general formula I;*

*Compound No. 3; wherein $Y=CH_3$, Z=ethynyl in the general formula I;*

*Compound No. 4; wherein $Y=CH_3$, Z=cyano in the general formula I; and*

*Compound No. 5; wherein $Y=CH_3$, Z=2-sulfono-5-methyl-1,3,4-thiadiazole in the general formula I.*

This experiment shows that compound 5, one of the derivatives of the general formula I, has significant activity in inhibiting the cytopathogenicity of HIV against T4 lymphocytes in-vitro.

A second experiment shown in Table II herebelow was conducted to show the effects of various compounds on replication of Vero cells. These cells are fast growing, and it can be seen that compound 5 exhibit little inhibition of these cells. This is indicative of much lower toxicity than AZT, (Compound No. 1) which inhibit a much higher percentage of these cells at a given concentration.

TABLE II

Effect of compound 5 on the replication of Vero Cells. [For methods, see Schinazi et al., Antimicrob. Agents Chemother. 22, 499-507 (1982)].

| Compound | Conc. (ug/ml) | Vero cells % inhibition |
|---|---|---|
| No. 5 | 50 | 0 |
| | 75 | 10 |
| | 100 | 20 |
| No. 1 (AZT) | 10 | 21 |
| | 100 | 86 |

The reader's attention is directed to all papers and documents which are filed concurrently with this specification, and which are open to public inspection with this specification and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in the specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A 1-(3′-substituted-2′,3′-dehydro-2′,3′-dideoxyribofuranosyl) pyrimidine derivative of the general formula I:

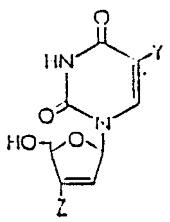

I

wherein Y is H, or a $C_1$ - $C_6$ alkyl group; and
Z is an azido group, or a 1-(1,2,3-triazolo) group or a cyano group, or an optionally substituted alkynyl group, or an optionally substituted alkenyl group, or an optionally substituted sulfide group or an optionally substituted sulfone group.

2. The derivative of formula I as set forth in claim 1 wherein Y is H or a $C_1$ - $C_6$ alkyl group and wherein Z is selected from an azido group, or a 1-(1,2,3-triazolo) group, or a cyano group or an optionally substituted alkynyl group or an optionally substituted alkenyl group wherein the optional substituents for the alkynyl group and alkenyl group are selected from H, or a $C_1$-$C_6$ alkyl group or a phenyl group, or an optionally substituted sulfide group or an optionally substituted sulfone group wherein the optional substituents for the sulfide group and sulfone group are selected from a cyano group, or a lower alkyl group, or a phenyl group or a heterocyclic moiety.

3. The derivative of formula I as set forth in claim 2 wherein the heterocyclic moiety is selected from the group comprising: 2-pyrimidinyl, 2-pyridinyl, 2-(1-methyl)imidazolo, 5-(1-methyl)tetrazolo, 2-benzimidazolo, 2-benzoxazolo, 2-benzthiazolo, 2-(5-methyl)-1,3,4-thiadiazolo, 3-1H-1,2,4-triazolo, 4-2H-1,2,3-triazolo, 2-(4-methyl)thiazole, or 2-thiazolinyl.

4. The derivative of formula I as set forth in any preceding claim 3 wherein Y is selected from H or $CH_3$ and Z is sulfone group optionally substituted by a cyano group, or a lower alkyl group or a phenyl group or a heterocyclic moiety.

5. A derivative as set forth in any preceding claim, selected from the following;
1-(2′,3′-didehydro-2′,3′-dideoxy-3′-(2″-sulfono-5″-methyl-1″,3″,4″-thiadiazolo)ribofuranosyl thymine;
1-(3′-azido-2′,3′-didehydro-2′,3′-dideoxyribofuranosyl)thymine;
1-(2′,3′-didehydro-2′,3′-dideoxy-3′-(1″-1″,2″,3″-triazolo)ribofuranosyl)thymine;
1-(2′,3′-didehydro-2′,3′-dideoxy-3′,-ethynylribofuranosyl)thymine;
1-(2′,3′-didehydro-2′,3′-dideoxy-3′-(2″-sulfono-4″-methylthiazolo)ribofuranosyl)thymine;
1-(2′,3′-didehydro-2′,3′-didehydro-3′-cyanoribofuranosyl)thymine; and
2′,3′-didehydro-2′,3′-dideoxy-3′-(2″-sulfono-5″-methyl)thiadiazolo)uridine;
2′,3′-didehydro-2′,3′-dideoxy-3′-ethynyluridine;
2′,3′-didehydro-2′,3′-dideoxy-3′-cyanouridine;

6. 1-[2′,3′-didehydro-2′,3′-dideoxy-3′-(2″-sulfono-5″-methyl-1″,3″,4″-thiadiazole)ribofuranosyl-]thymine.

7. A derivative of the general formula III:

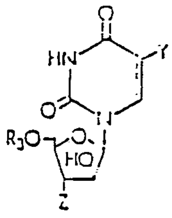 ...III

wherein Y is H or a $C_1$ - $C_6$ alkyl group;
Z is an azido group, or a cyano group, or a 1-(1,2,3-triazole group), or an optionally substituted alkynyl group, or an optionally substituted alkenyl group, or an optionally substituted sulfide group or an optionally substituted sulfone group; and
$R_3$ is a protecting group selected from the group comprising trityl, dimethoxytrityl, trimethylsilyl, t-butyl dimethylsilyl, p-nitrobenzoyl, benzoyl or an acetyl group.

8. A derivative of the general formula IV:

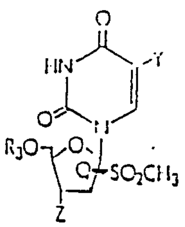 ...IV

wherein Y is H or a $C_1$-$C_6$ alkyl group;
Z is an azido group, or a cyano group, or a 1-(1,2,3-triazolo group), or an optionally substituted alkynyl group, or an optionally substituted alkenyl group, or an optionally substituted sulfide group, or an optionally substituted sulfone group; and
$R_3$ is a protecting group selected from the group comprising trityl, dimethoxytrityl, trimethylsilyl, t-butyldimethylsiyl, p-nitrobenzoyl, benzoyl or an acetyl group.

9. A derivative of the general formula V:

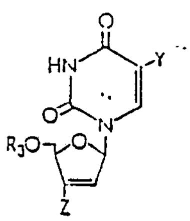 ...V

wherein Y is H or a $C_1$-$C_6$ alkyl group;
Z is an azido group, or a cyano group, or a 1-(1,2,3-triazolo group), or an optionally substituted alkynyl group, or an optionally substituted alkenyl group, or an optionally substituted sulfide group, or an optionally substituted sulfone group; and
$R_3$is a protecting group selected from the group comprising trityl, dimethoxytrityl, trimethylsilyl, t-butyldimethylsiyl, p-nitrobenzoyl, benzoyl or an acetyl group.

10. A process substantially as set forth herein, for the preparation of a derivative of formula I, as defined in any of claims 1 to 6; or for the preparation of an intermediate therefor, as described or claimed herein.

11. A pharmaceutical composition comprising a compound as set forth in any of claims 1 to 6, together with a pharmaceutically acceptable carrier.

12. A compound as set forth in any of claims 1 to 6, for use as a therapeutic agent.

13. A method of combating the HIV virus comprising treating the virus with a compound of any of claims 1 to 6.

II. III. IV.

V.

I.

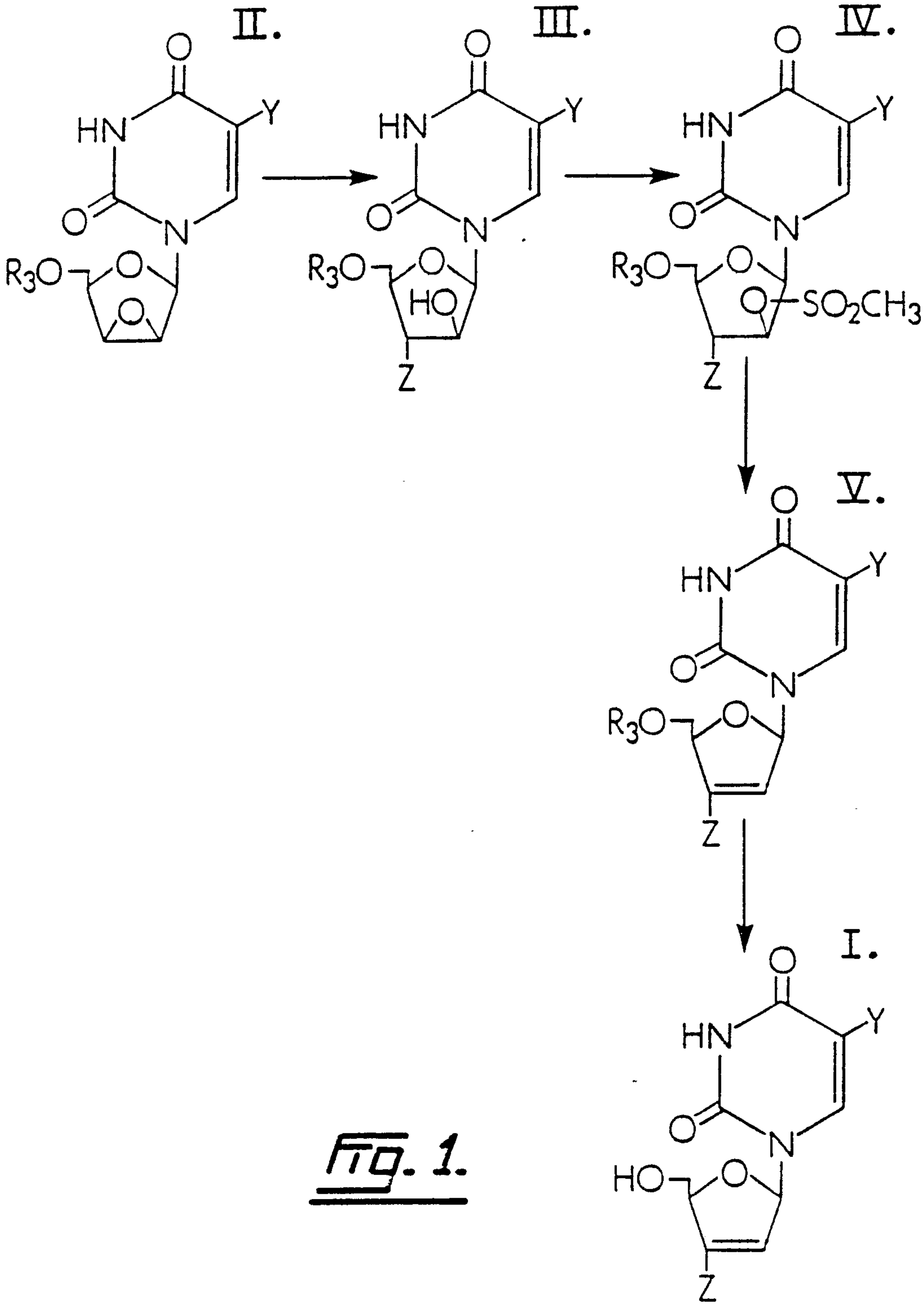

Fig. 1.

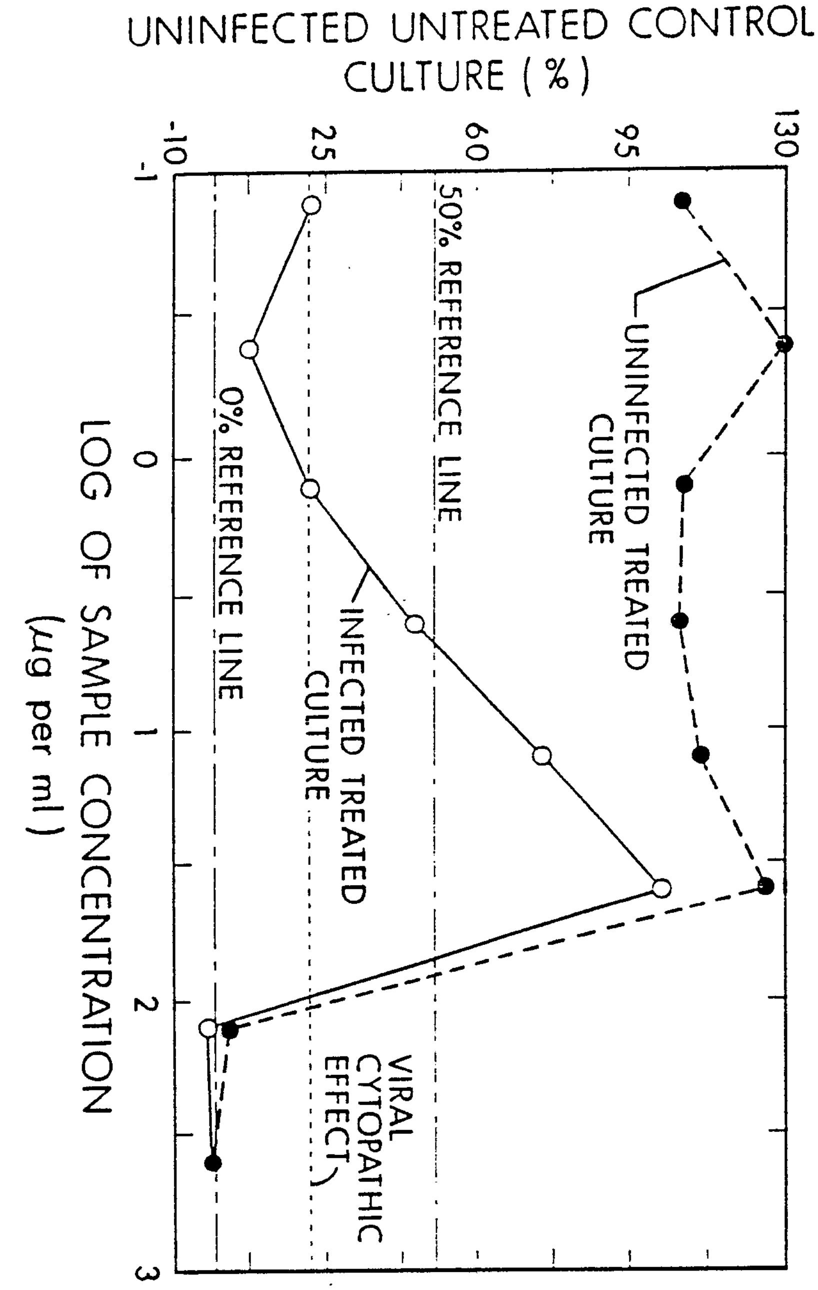
UNINFECTED UNTREATED CONTROL CULTURE (%)
130
95
60
25
-10
-1
0
1
2
3
UNINFECTED TREATED CULTURE
50% REFERENCE LINE
INFECTED TREATED CULTURE
0% REFERENCE LINE
VIRAL CYTOPATHIC EFFECT
LOG OF SAMPLE CONCENTRATION
(μg per ml)

Fig. 2.